Europäisches Patentamt

European Patent Office

Office européen des brevets

(11) Veröffentlichungsnummer: **0 237 589**
**A1**

## (12) EUROPÄISCHE PATENTANMELDUNG

(21) Anmeldenummer: 86103516.0

(22) Anmeldetag: 15.03.86

(51) Int. Cl.4: **A61F 13/20** , A61F 11/00

(43) Veröffentlichungstag der Anmeldung:
**23.09.87 Patentblatt 87/39**

(84) Benannte Vertragsstaaten:
**AT BE CH DE FR GB IT LI LU NL SE**

(71) Anmelder: **Fassbind & Fassbind**
**Appisbergstrasse 20**
**CH-8708 Männedorf(CH)**

(72) Erfinder: **Fassbind, Karl**
**Appisbergstrasse 20**
**CH-8708 Männedorf(CH)**

(74) Vertreter: **Blum, Rudolf Emil Ernst et al**
**c/o E. Blum & Co Patentanwälte Vorderberg**
**11**
**CH-8044 Zürich(CH)**

(54) **Wattestäbchen.**

(57) Damit das Wattestäbchen beim Reinigen des Gehörganges nicht zu tief in diesen eindringen kann, ist das Wattepolster (2) mit einem Anschlag (9) versehen. An diesen schliesst sich eine zylindrische Kopfpartie (3,7) an, mit der der Gehörgang durch Drehen des Wattestäbchens ausgerieben und damit gereinigt werden kann. Zum Aufrechterhalten der Formstabilität der zylindrischen Kopfpartie (3,7) an sich und auch bezügliche der dazu quer liegenden Fläche des Anschlages (9) ragt der Tragschaft -(1,6,4) des Wattestäbchens in die zylindrische Kopfpartie (3,7) und bildet darin einen stabilen Kern.

# Fig.1

EP 0 237 589 A1

## Wattestäbchen

Die Erfindung betrifft ein Wattestäbchen nach dem Oberbegriff des Patentanspruchs 1.

Ein solches bekanntes Wattestäbchen ist durch die europäische Patentanmeldung mit der Veröffentlichungsnummer 120 235 sowie die US-PS 2 510 961 und 2 876 501 bekannt. Bei diesen bekannten Wattestäbchen kann die im Durchmesser grössere Bauchpartie des Wattepolsters eine gewisse Bremswirkung beim Einführen des Wattestäbchens in den Gehörgang ausüben. Durch diese Bremswirkung soll einem zu tiefen Eindringen des Wattestäbchens in den Gehörgang entgegengewirkt werden. Die erwähnte mehr oder weniger stark wirkende Bremswirkung kann aber nicht einwandfrei verhindern, dass das Wattestäbchen doch zu tief in den Gehörgang eindringt, das Trommelfell verletzen kann und auch Ohrenschmalz tief ins Innere des Gehörgangs geschoben wird. Durch dieses tiefe Einschieben des Ohrenschmalzes kann die Hörfähigkeit nahezu aufgehoben werden und weiterhin können Gleichgewichtsstörungen auftreten. Es wurde also oft festgestellt, dass nach einer Manipulation im Gehörgang mittels eines bekannten Wattestäbchens ein nach innen verschobener Ohrenschmalzpfropfen vom Arzt durch Wasserspülungen mit verhältnismässig hohem Druck herausgespült werden musste. Es wurden weiterhin schon oft Verletzungen des inneren Gehörganges durch zu tiefes Eindringen des Wattepolsters in den Gehörgang festgestellt.

Es wird die Schaffung eines Wattestäbchens bezweckt, mit dem die vorerwähnten Nachteile vermieden werden können. Weiterhin soll das zu - schaffende Wattestäbchen möglichst wirtschaftlich hergestellt werden können, wobei hierzu auch, bei ungeschmälerter Wirkung, möglichst wenig Material pro Wattestäbchen aufgewandt werden soll. Weiterhin soll das zu schaffende Wattestäbchen eine möglichst hohe Formstabilität aufweisen, damit es seine bei der Herstellung am zweckmässigen gewählte Form auch bei intensivstem Gebrauch beibehält.

Die erfindungsgemässe Ausbildung des Wattestäbchens ergibt sich aus dem kennzeichnenden Teil des Patentanspruchs 1.

Durch den Anschlag wird nunmehr genau die Länge des Wattepolsters bestimmt, die zum Eindringen in den Gehörgang zur Verfügung steht. Diese Eindringtiefe wird auch durch mehrmaliges Drehen des Wattestäbchens im Gehörgang nicht verändert, wobei mit dem zylindrischen Abschnitt der Gehörgang durch Drehen des Wattestäbchens ausgerieben und damit gereinigt wird. Gleichzeitig wird mit der Fläche des Anschlags der Uebergang von der Ohrmuschel zum Gehörgang gereinigt.

Dadurch, dass sich der Tragschaft teilweise in die Kopfpartie des Wattepolsters hineinerstreckt, dient das Tragschaftende als stabiler Kern des zylindrischen Abschnitts, so dass dieser formstabil, d.h. parallel zum Tragschaft verbleit, also nicht gegenüber diesem abgebogen werden kann.

In der Zeichnung sind drei Aufführungsbeispiele des Erfindungsgegenstandes dargestellt. Es zeigen:

Fig. 1 das Ende eines Wattestäbchens mit einem Wattepolster, in einer ersten Ausführungsform der Bauchpartie, in Seitenansicht,

Fig. 2 eine zweite Ausführungsform der Bauchpartie, ebenfalls in Seitenansicht, und

Fig. 3 eine dritte Ausführungsform der Bauchpartie, im Längsschnitt.

Das Wattestäbchen hat einen Tragschaft 1, der an zumindest einem Ende mit einem Wattepolster 2 versehen ist. Das Wattepolster 2 hat einem im Durchmesser kleinere Kopfpartie 3, die beim Tragschaftende 4 liegt, und eine im Durchmesser grössere Bauchpartie 5, die dem mittleren Bereich 6 des Tragschaftes 1 zugewandt ist. Der Tragschaft 1 durchsetzt die Bauchpartie 5. Die Kopfpartie 3 umfasst einen zylindrischen Abschnitt 7 und einen balligen Abschnitt 8, wobei der ballige Abschnitt 8 stufenlos in den zylindrischen Abschnitt übergeht. Die Bauchpartie 5 hat eine an den zylindrischen Abschnitt 7 angrenzende und als Anschlag für diesen Abschnitt 7 dienende Ringfläche 9. Der Tragschaft 1 überragt diese Ringfläche 9 und erstreckt sich in den zylindrischen Abschnitt 7 hinein. Der Tragschaft 1 liegt parallel zum zylindrischen Abschnitt 7. Bei den dargestellten Ausführungsbeispielen erstreckt sich der Tragschaft 1 über den gesamten zylindrischen Abschnitt 7, so dass also die ballige Kontur des Abschnitts 8 um das Mass seines Radius vom Tragschaftende 4 distanziert liegt.

Die Bauteile 3, 4, 7 und 8 sind bei allen drei Ausführungsbeispielen gleichen ausgebildet. Die Unterschiede zwischen den drei Ausführungsbeispielen liegen lediglich in der verschiedenen Ausbildung der Bauchpartie 5 und der radialen Erstreckung der Ringfläche 9.

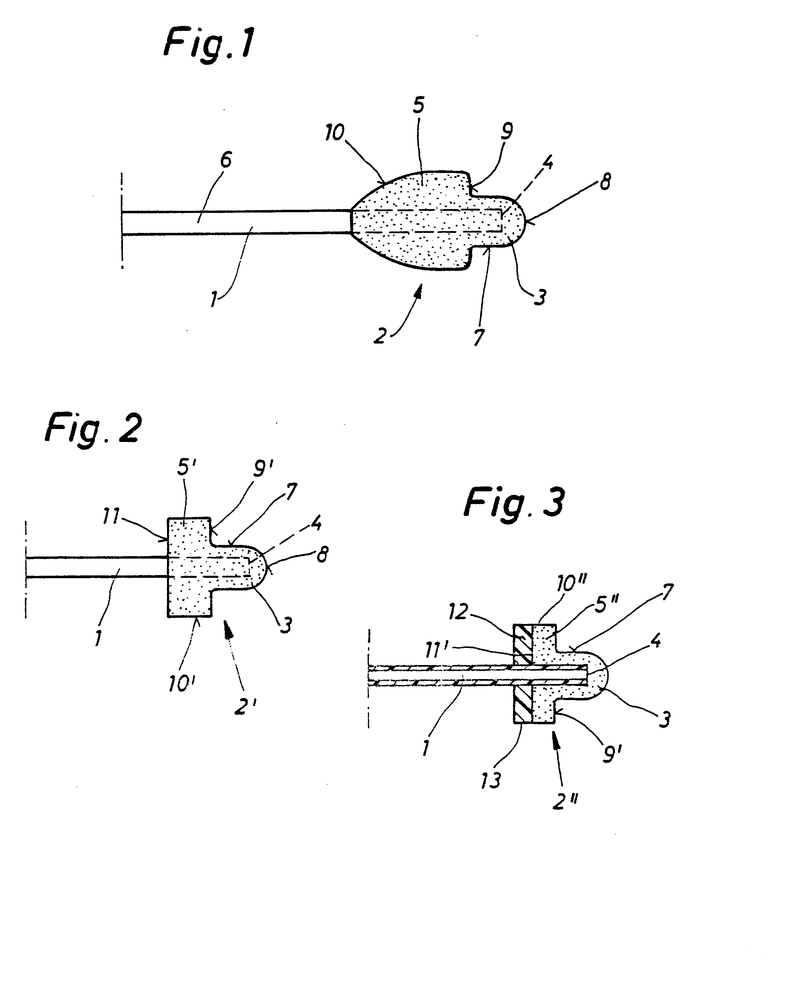

Beim Beispiel nach Fig. 1 hat die Bauchpartie 5 neben der Ringfläche 9 eine ballige Kontur 10, die von der Ringfläche 9 bis zur Mantelfläche des Tragschaftes 1 führt.

Beim Beispiel nach Fig. 2 hat die Bauchpartie 5' neben ihrer Ringfläche 9' einen Zylindermantel 10' sowie eine Planfläche 11. Diese Planfläche 11 liegt um die Länge der Bauchpartie 5' im Abstand zur Ringfläche 9'. Bei diesem in Fig. 2 gezeigten Ausführungsbeispiel wird weniger Watte für das

Wattepolster 2' als für das Wattepolster 2 nach Fig. 1 benötigt. Da bei der Herstellung des Wattestäbchens nach Fig. 2 an der Planfläche 11 ein nicht dargestelltes Werkzeug anliegt, kann dieses als Gegenhalter (Amboss) für ein an der Ringfläche 9' angreifendes Werkzeug dienen, so dass hierdurch die Ringfläche 9' besser verfestigt werden kann als beim Beispiel nach Fig. 1.Auf die Ringfläche 9' kann also mit einem einfacheren Werkzeug eine grössere Verfestigungskraft auf die Watte ausgeübt werden.

Beim Beispiel nach Fig. 3 wird noch weniger Watte benötigt, da die Bauchpartie 5″ des Wattepolsters 2″ kürzer als beim Beispiel nach Fig. 2 ausgebildet ist. Damit diese Bauchpartie 5″ genügend stabil ist, ist sie mit einer Kunststoffscheibe 12 versehen. Diese haftet an der Planfläche 11' der Bauchpartie 5″ an und ist zum Beispiel daran angeklebt. Bei einer möglichen Ausführungsform braucht die Kunststoffscheibe 12 am Tragschaft 1 selbst gar nicht befestigt zu werden, sondern sitzt auf dieser spielfrei. Die Kunststoffscheibe 12 hat eine zylindrische Mantelfläche 13, die im Durchmesser gleich ist wie die zylindrische Mantelfläche 10″ des Wattepolsters.

Bei allen drei Ausführungsformen liegt die Ringfläche 9, 9' quer zwischen der Bauchpartie 5', 5″ und der Kopfpartie 3. Weiterhin ist bei allen drei Ausführungsformen der Durchmesser der Bauchpartie 5, 5' und 5″ im wesentlichen doppelt so gross wie der Durchmesser der Kopfpartie 3. Bei allen Ausführungsbeispielen hat die Ringfläche 9 bzw. 9' einen inneren Durchmesser, der dem der Kopfpartie 3 entspricht und weiterhin einen äusseren Durchmesser, der dem der Bauchpartie 5, 5' oder 5″ entspricht. Bei allen drei Ausführungsbeispielen steht die Ringfläche 9 bzw. 9' recht winklig zur Längsachse des Tragschaftes 1. Bei den beiden Ausführungsformen nach den Fig. 2 und 3 liegen die Planfläche 11 bzw. 11' und die Ringfläche 9' parallel zueinander.

Der Tragschaft 1 besteht in bekannter Weise aus einem verhältnismässig stabilen Kunststoffrohr, Holz-oder Papierschaft. Da das Tragschaftende 4 somit als stabiler Kern radial innerhalb des zylindrischen Abschnittes 7 liegt und auch die gesamte Bauchpartie 5, 5' oder 5″ durchragt, erhält das Wattepolster eine sehr stabile Form, die auch bei intensiver Benutzung des Wattestäbchens beibehalten wird. Hierdurch kann der Gehörgang mit dem zylindrischen Abschnitt 7 durch Drehen des Wattestäbchens gut ausgerieben und damit gereinigt werden. Gleichzeitig ergibt die Ringfläche 9, 9' einen formstabilen Anschlag am Uebergang zwischen Ohrmuschel und Gehörgang. Wie bereits erwähnt, kann diese Ringfläche 9 bzw. 9' des Anschlags eine sehr gute Verfestigung der Watte aufweisen, die der Formstabilität des Wattepolsters zugute kommt.

## Ansprüche

1. Wattestäbchen, bei dem ein Tragschaft (1) an zumindest einem Ende mit einem Wattepolster (2,2',2″) versehen ist, wobei eine im Durchmesser kleinere Kopfpartie (3) des Wattepolsters beim Tragschaftende (4) liegt und eine im Durchmesser grössere Bauchpartie des Wattepolsters (5,5',5″) dem mittleren Bereich (6) des Tragschaftes (1) zugewandt ist, wobei der Tragschaft (1) die Bauchpartie (5,5',5″) durchsetzt, dadurch gekennzeichnet, dass die Kopfpartie (3) einen zylindrischen Abschnitt (7) umfasst, dass die Bauchpartie (5,5',5″) eine an den zylindrischen Abschnitt (7) angrenzende und als Anschlag für diesen Abschnitt dienende Ringfläche (9, 9') aufweist, und dass der Tragschaft (1) diese Ringfläche (9,9') überragt und sich in den zylindrischen Abschnitt (7) hinein erstreckt und parallel zu diesem liegt.

2. Wattestäbchen nach Anspruch 1, dadurch gekennzeichnet, dass die Ringfläche (9,9') quer zwischen der Bauchpartie (5,5',5″) und der Kopfpartie (3) liegt.

3. Wattestäbchen nach Anspruch 2, dadurch gekennzeichnet, dass die Ringfläche (9,9') rechtwinklig zur Längsachse des Tragschaftes (1) steht.

4. Wattestäbchen nach Anspruch 1, dadurch gekennzeichnet, dass der Durchmesser der Bauchpartie (5,5',5″) im wesentlichen doppelt so gross ist wie der Durchmesser der Kopfpartie (3).

5. Wattestäbchen nach Anspruch 1, dadurch gekennzeichnet, dass die Ringfläche (9,9') einen inneren Durchmesser hat, der dem der Kopfpartie (3) entspricht und weiterhin einen äusseren Durchmesser hat, der dem der Bauchpartie (5,5',5″) entspricht.

6. Wattestäbchen nach Anspruch 1, dadurch gekennzeichnet, dass die Bauchpartie (5') eine Planfläche (11) aufweist, die um die Länge der Bauchpartie im Abstand zur Ringfläche (9') liegt (Fig. 2).

7. Wattestäbchen nach Anspruch 6, dadurch gekennzeichnet, dass Planfläche (11,11') und Ringfläche (9') zueinander parallel liegen (Fig. 2,3).

8. Wattestäbchen nach Anspruch 6, dadurch gekennzeichnet, dass an der Planfläche (11') eine Kunststoffscheibe (12) befestigt ist, die auf dem Tragschaft (1) sitzt (Fig. 3).

# Fig.1

# Fig. 2

# Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 120 235  (FASSBIND & FASSBIND) <br> * Insgesamt * | 1-7 | A 61 F  13/20 <br> A 61 F  11/00 |
| | --- | | |
| Y | DE-C- 604 145  (STILLER) <br> * Abbildung 1;  Seite  1,  Zeilen 28-44 * | 1-7 | |
| | --- | | |
| A | US-A-1 980 826  (REISS) <br> * Abbildung 4; Seite 1, Zeile 94 - Seite 2, Zeile 14 * | 8 | |
| | --- | | |
| A | US-A-3 818 911  (FOURNIER) | | |
| | ----- | | |

| RECHERCHIERTE SACHGEBIETE (Int. Cl.4) |
|---|
| A 61 F <br> A 45 D |

Der vorliegende Recherchenbericht wurde für alle Patentansprüche erstellt.

| Recherchenort <br> DEN HAAG | Abschlußdatum der Recherche <br> 01-09-1986 | Prüfer <br> STEENBAKKER J. |
|---|---|---|

KATEGORIE DER GENANNTEN DOKUMENTE
X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder
nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82